# EUROPEAN PATENT APPLICATION

(11) **EP 4 623 894 A1**
(43) Date of publication of application: **01.10.2025**
(21) Application number: 23894079.5
(22) Date of filing: 21.11.2023
(51) Int. Cl.: A61K 8/44, A61K 8/34, A61K 8/36, A61K 8/41, A61Q 19/10, C11D 1/04, C11D 1/10, C11D 3/30

(54) **SKIN CLEANSER COMPOSITION**

(30) Priority: 24.11.2022 CN 202211481938
(71) Applicant: Kao Corporation, Chuo-ku, Tokyo 103-8210 (JP)
(72) Inventor: LI, Zeqian, Shanghai 200241 (CN); UMEHARA, Kaori, Tokyo 131-0044 (JP); FURUKAWA, Kurenai, Tokyo 131-0044 (JP); SUN, Zhirong, Shanghai 200241 (CN); LIU, Yanqing, Shanghai 200241 (CN)
(74) Representative: Hoffmann Eitle
(86) International application number: PCT/IB2023/061718
(87) International publication number: WO 2024/110854

(57) **Abstract**

The present invention relates to a skin cleanser composition containing the following components (A)-(B): component (A) is one or more among an N-acyl amino acid represented by formula (1) or a salt thereof (A1), an N-acyl taurine represented by formula (2) or a salt thereof (A2), and a fatty acid having 8-20 carbon atoms or a salt thereof (A3); and component (B) is one or more among TRIS, AMP, AMPD, and MEA. (In the formulae, R¹ is a C5-24 linear or branched alkyl or alkenyl group, R² is hydrogen or a C1-4 alkyl group, R³ and R⁴ are each hydrogen, a C1-4 alkyl group, a C1-4 hydroxyalkyl group or -(CH ₂)ₘ₁-COOM², m1 and n1 are numbers from 0-20, M¹ and M² are each hydrogen, an alkali metal, an alkaline earth metal, a basic amino acid, ammonium, or an alkanolamine; R₁ is a C8-20 linear or branched alkyl or alkenyl group, R₂ is hydrogen or C1-3 alkyl group, and M is hydrogen, an alkali metal, an alkaline earth metal, an basic amino acid, ammonium, or an alkanolamine.)

## Description

### Field of the Invention

The present invention relates to a skin cleanser composition.

### Background of the Invention

Dirt in pores is one of the main cleaning targets in skin cleansing. The dirt in the pores mainly includes oil dirt, makeup cosmetic dirt, and keratotic plugs. Among them, the keratotic plugs are commonly observed in the pores at sites with increased sebum secretion such as around the nose, and are plug-shaped dirt that clogs the pores. The keratotic plugs are mainly composed of proteins, lipids etc.

If the keratotic plugs are left without being removed, the skin will become rough. At the same time, the keratotic plugs physically expand the pores, rendering the pores to become large. Additionally, oxidation of the surfaces of the keratotic plugs may lead to blackhead formation, leading to spoiling the beauty appearance. Moreover, these may cause acne, etc., to readily result in skin troubles such as rashes and pimples. Due to these facts, the keratotic plugs become one of the reasons why the pores are obvious, and have become a cause of trouble for many women. Therefore, there is a need for a variety of skin cleansers that can effectively remove dirt from pores, particularly keratotic plugs.

For instance, Patent Literature 1 discloses a method for fully exerting an excellent effect of removing keratotic plugs without causing any burden such as pain or irritation to the skin. The method removes keratotic plugs by using a composition comprising one or more selected from 2-amino-2-hydroxymethyl-1,3-propanediol, 2-amino-2-methyl-1-propanol, and 2-amino-2-methyl-1,3-propanediol. It is further disclosed that the composition may further comprise an anionic surfactant to enhance foaming property.

Patent Literature 1: International Patent Publication No. WO2018/190301

### Summary of the Invention

However, in the composition described in Patent Literature 1, if the component with the effect of removing keratotic plugs, such as 2-amino-2-hydroxymethyl-1,3-propanediol, is used in combination with a large amount of any anionic surfactant, it will cause difficulties in balancing foaming property and the detergency against keratotic plugs. Furthermore, if the cleanser composition disclosed in Patent Literature 1 is put into a foam discharge container for use, components with keratotic plugs-removing effect, such as 2-amino-2-hydroxymethyl-1,3-propanediol, are prone to crystallize and precipitate over time, leading to clogging of the nozzle of the foam discharge container, thereby causing a problem that foam cannot be stably discharged from the foam discharge container for a long period of time.

**In** view of the technical problem existing in the prior art mentioned above, the inventors have conducted diligent studies and have found that by using a specific anionic surfactant in combination with a component having keratotic plugs-removing effect, a skin cleanser composition that achieves both excellent detergency against pores, particularly the detergency against keratotic plugs, and excellent foaming property could be obtained. Thus, the invention has been completed.

The present invention provides a skin cleanser composition comprising the following component (A) and component (B),
component (A) is one or more anionic surfactant(s) selected from the following components (A1), (A2), and (A3),
component (A1) is an N-acyl amino acid represented by the following general formula (1), or a salt thereof; 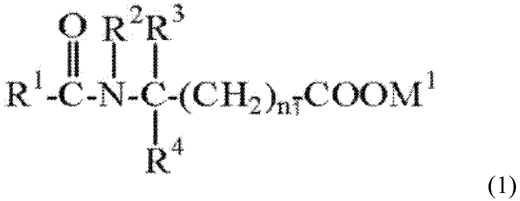
(In formula (1), R¹ represents a linear or branched alkyl or alkenyl with 5 to 24 carbon atoms. R² represents hydrogen or an alkyl with 1 to 4 carbon atoms. R³ and R⁴ are the same or different and represent hydrogen, an alkyl with 1 to 4 carbon atoms, a hydroxyalkyl with 1 to 4 carbon atoms, or -(CH₂)ₘ₁-COOM². m1 and n1 are each independently a number from 0 to 20. M¹ and M² are the same or different and represent hydrogen, an alkali metal, an alkaline earth metal, a basic amino acid, ammonium or alkanolamine.)
component (A2) is an N-acyl taurine represented by the following general formula (2), or a salt thereof;

   R₁CO-NR₂-CH₂CH₂SO₃M (2)
(In formula (2), R¹ represents a linear or branched alkyl or alkenyl with 8 to 20 carbon atoms. R₂ represents hydrogen or an alkyl with 1 to 3 carbon atoms. M represents hydrogen, an alkali metal, an alkaline earth metal, a basic amino acid, ammonium, or alkanolamine.)
component (A3) is a fatty acid with 8 to 20 carbon atoms, or a salt thereof;
component (B) is one or more selected from 2-amino-2-hydroxymethyl-1,3-propanediol, 2-amino-2-methyl-1-propanol, 2-amino-2-methyl-1,3-propanediol, and monoethanolamine.

Furthermore, the inventors have discovered that by further comprising polyol(s) with a specific amount in the aforementioned skin cleanser composition, it is possible to not only achieve excellent detergency against pores, excellent foaming property, as well as foam quality, but also inhibit the formation of crystallization of components with keratotic plugs-removing effect, thereby preventing the clogging of the foam discharge container.

In addition, the present invention also provides a foam discharge container-packed skin cleanser composition, which comprises the above-mentioned component (A), component (B) and polyol(s).

### Effect of the invention

According to the present invention, a skin cleanser composition that achieves both excellent detergency against pores, particularly detergency against keratotic plugs, and excellent foaming property can be provided. Furthermore, the present invention also provides a foam discharge container-packed skin cleanser composition which not only achieves excellent detergency against pores, particularly detergency against keratotic plugs, as well as excellent foaming property and foam quality, but also prevents the clogging of the foam discharge container.

### Detailed Description of the Invention

In the present specification, the term "keratotic plug" refers to a substance physiologically formed in the pores of human skin and occluding the pores in a plug-like manner. The main components of the keratotic plug are proteins derived from the stratum corneum and hair follicles. The keratotic plug is formed of a material in which these main components are mixed with proteins including proteins derived from Propionibacterium acnes and Staphylococcus aureus or proteins derived from subcellular organelles such as lysosomes, and lipids including triglyceride, free fatty acids or peroxidized lipids.

Hereinafter, each component comprised in the skin cleanser composition of the present invention is described in detail.

### <Component (A)>

As the component (A), one or more anionic surfactant(s) selected from components (A1), (A2), and (A3) can be used, from the viewpoint of further achieving both excellent detergency against pores, detergency against keratotic plugs, and excellent foaming property, it is preferred to use one or more selected from component (A1) and component (A2), and more preferably one or more selected from component (A1).

Components (A1) to (A3) are described below.

### (Component (A1))

The component (A1) used in the skin cleanser composition of the present invention is an N-acyl amino acid represented by the following general formula (1), or a salt thereof.

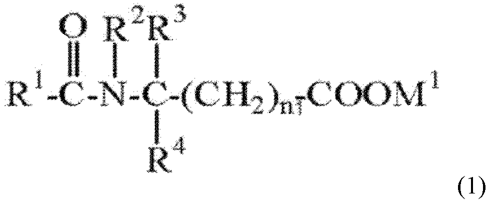

(In formula (1), R¹ represents a linear or branched alkyl or alkenyl with 5 to 24 carbon atoms. R² represents hydrogen or an alkyl with 1 to 4 carbon atoms. R³ and R⁴ are the same or different and represent hydrogen, an alkyl with 1 to 4 carbon atoms, a hydroxyalkyl with 1 to 4 carbon atoms, or -(CH₂)ₘ₁-COOM². m1 and n1 are each independently a number from 0 to 20. M¹ and M² are the same or different and represent hydrogen, an alkali metal, an alkaline earth metal, a basic amino acid, ammonium or alkanolamine.)

In the above formula (1), from the viewpoint of further achieving both excellent detergency against pores, detergency against keratotic plugs, and excellent foaming property, R¹ is preferably a linear or branched alkyl or alkenyl with 8 to 20 carbon atoms, more preferably a linear or branched alkyl with 8 to 20 carbon atoms, and further preferably a linear or branched alkyl with 8 to 18 carbon atoms.

In addition, from the viewpoint of further achieving both excellent detergency against pores, detergency against keratotic plugs, and excellent foaming property, R² is preferably an alkyl with 1 to 3 carbon atoms, more preferably methyl or ethyl, and further preferably methyl.

In addition, from the viewpoint of further achieving both excellent detergency against pores, detergency against keratotic plugs, and excellent foaming property, R³ and R⁴ are each preferably an alkyl with 1 to 4 carbon atoms, a hydroxyalkyl with 1 to 4 carbon atoms, or -(CH₂)ₘ₁-COOM², more preferably an alkyl with 1 to 4 carbon atoms, -(CH₂)ₘ₁-COOM², and further preferably -(CH₂)ₘ₁-COOM².

In addition, examples of M¹, M² include hydrogen; alkali metals such as sodium and potassium; alkaline earth metals such as calcium and magnesium; ammonium; alkanolamines such as monoethanolamine, diethanolamine, and triethanolamine; or cations from basic amino acids such as arginine and lysine.

Examples of the acyl of the N-acyl amino acid or a salt thereof used in the present invention include acyl groups derived from fatty acids such as caprylic acid, capric acid, lauric acid, myristic acid, palmitic acid, stearic acid, oleic acid, and behenic acid. Among these fatty acids, from the viewpoint of further achieving both excellent detergency against pores, detergency against keratotic plugs, and excellent foaming property, it is preferably one or more selected from lauric acid, myristic acid, palmitic acid, and oleic acid, more preferably lauric acid. In addition, the acyl of the N-acyl amino acid or a salt thereof can also be derived from a mixed fatty acid of the above-mentioned fatty acids, for example, it can also be derived from a mixed fatty acid obtained by using coconut oil, palm kernel oil, etc. as a raw material, wherein it is preferably an acyl obtained by using coconut oil fatty acid or palm kernel oil fatty acid as a raw material, and more preferably an acyl obtained by using coconut oil fatty acid as a raw material.

Examples of the N-acyl amino acid or salt thereof used in the present invention include N-acyl glycine or a salt thereof, N-acyl alanine or a salt thereof, N-acyl threonine or a salt thereof, N-acyl serine or a salt thereof, N-acyl sarcosine or a salt thereof, N-acyl glutamic acid or a salt thereof, N-acyl aspartic acid or a salt thereof, and the like. Among them, from the viewpoint of further achieving excellent detergency against pores, detergency against keratotic plugs, and excellent foaming property, it is preferably N-acyl amino acid with a branched structure or a salt thereof, more preferably N-acyl alanine or a salt thereof, N-acyl glutamic acid or a salt thereof, N-acyl threonine or a salt thereof, N-acyl serine or a salt thereof, and N-acyl aspartic acid or a salt thereof, and further preferably N-acyl glutamic acid or a salt thereof, and N-acyl aspartic acid or a salt thereof.

As the salt of the above-mentioned N-acyl amino acid or salt thereof, from the viewpoint of reducing irritation to the skin or from the viewpoint of easiness of acquisition, it is preferably one or more salts selected from an alkali metal salt, a triethanolamine salt and an arginine salt, more preferably one or more salts selected from a sodium salt, a potassium salt, an arginine salt and a triethanolamine salt, further preferably one or more salts selected from a sodium salt and a potassium salt, and further preferably a sodium salt.

The above-mentioned N-acyl amino acid or salt thereof can be used either individually or in combination of two or more.

When the skin cleanser composition of the present invention comprises the component (A1), from the viewpoint of achieving both excellent detergency against pores, detergency against keratotic plugs, and excellent foaming property, the content of the component (A1) in terms of acid is preferably 0.5% by mass or more, more preferably 0.8% by mass or more, and further preferably 1% by mass or more. Additionally, from the viewpoint of preventing an increase in burden on the skin as well as achieving both excellent detergency against pores, detergency against keratotic plugs, and excellent foaming property, the content of the component (A1) in terms of acid is preferably 15% by mass or less, 7.5% by mass or less, further preferably 5% by mass or less, and further more preferably 4% by mass or less.

### (Component (A2))

The component (A2) used in the skin cleanser composition of the present invention is a N-acyl taurine represented by the following general formula (2), or a salt thereof.

R₁CO-NR₂-CH₂CH₂SO₃M (2)

(In formula (2), R¹ represents a linear or branched alkyl or alkenyl with 8 to 20 carbon atoms. R² represents hydrogen or an alkyl with 1 to 3 carbon atoms. M represents hydrogen, an alkali metal, an alkaline earth metal, a basic amino acid, ammonium, or alkanolamine.)

In the above formula (2), from the viewpoint of further achieving both excellent detergency against pores, particularly detergency against keratotic plugs, and excellent foaming property, R¹ is preferably a linear or branched alkyl or alkenyl with 8 to 18 carbon atoms, more preferably a linear or branched alkyl with 10 to 18 carbon atoms.

In addition, from the viewpoint of further achieving excellent detergency against pores, particularly detergency against keratotic plugs, and excellent foaming property, R² is preferably an alkyl with 1 to 3 carbon atoms, more preferably methyl or ethyl, and further preferably methyl.

In addition, examples of M include hydrogen; alkali metals such as sodium and potassium; alkaline earth metals such as calcium and magnesium; ammonium; alkanolamines such as monoethanolamine, diethanolamine, and triethanolamine; or cations derived from basic amino acids such as arginine and lysine. Among them, sodium and potassium are preferred.

Specific examples of such N-acyl taurine or salt thereof include, for example, N-lauroyl-N-methyltaurine or a salt thereof, N-myristoyl-N-methyltaurine or a salt thereof, N-cocoyl-N-methyltaurine or a salt thereof, N-lauroyl-N-ethyltaurine or a salt thereof, N-myristoyl-N-ethyltaurine or a salt thereof, N-cocoyl-N-ethyltaurine or a salt thereof, N-lauroyltaurine or a salt thereof, N-cocoyltaurine or a salt thereof, but are not limited to these.

The above-mentioned N-acyl taurine or salt thereof can be used either individually or in combination of two or more.

When the skin cleanser composition of the present invention comprises the component (A2), from the viewpoint of achieving both excellent detergency against pores, particularly detergency against keratotic plugs, and excellent foaming property, the content of the component (A2) in terms of acid is preferably 0.1% by mass or more, more preferably 0.4% by mass or more, further preferably 1.5% by mass or more, and further preferably 1.8% by mass or more. In addition, from the viewpoint of preventing an increase in burden on the skin and achieving both excellent detergency against pores, particularly detergency against keratotic plugs, and excellent foaming property, the content of the component (A2) in terms of acid is preferably 25% by mass or less, more preferably 23% by mass or less, further preferably 15% by mass or less, and further more preferably 6% by mass or less.

### (Component (A3))

The component (A3) used in the skin cleanser composition of the present invention is a fatty acid with 8 to 20 carbon atoms, or a salt thereof.

As fatty acids or salts thereof that can be used as the component (A3), from the viewpoint of further achieving excellent detergency against pores, particularly detergency against keratotic plugs, and excellent foaming property, it is preferably a fatty acid having a linear or branched alkyl with 10 to 20 carbon atoms or a salt thereof, more preferably a fatty acid having a linear or branched alkyl with 10 to 18 carbon atoms or a salt thereof, further preferably a fatty acid having a linear alkyl with 12 to 16 carbon atoms or a salt thereof, and further preferably a fatty acid having a linear alkyl with 12 to 14 carbon atoms or a salt thereof. Specifically, examples include one or more selected from lauric acid, myristic acid, palmitic acid, stearic acid, behenic acid, and salts thereof. Among them, from the same viewpoint as above, it is preferred to comprise one or more selected from lauric acid or a salt thereof, myristic acid or a salt thereof, and palmitic acid or a salt thereof, and more preferably one or two selected from lauric acid or a salt thereof, and myristic acid or a salt thereof. Examples of the salt of the fatty acid salt constituting the component (A3) include alkali metals such as sodium and potassium; ammonium; and basic amino acids such as arginine and lysine. Among them, sodium and potassium are preferred.

The above-mentioned fatty acids or salts thereof can be used either individually or in combination of two or more.

When the skin cleanser composition of the present invention comprises the component (A3), from the viewpoint of achieving both excellent detergency against pores, particularly detergency against keratotic plugs, and excellent foaming property, the content of the component (A3) in terms of acid in the skin cleanser composition is preferably 1% by mass or more, more preferably 2% by mass or more. In addition, from the viewpoint of preventing an increase in burden on the skin and exhibiting excellent detergency against pores, the content of the component (A3) in terms of acid in the skin cleanser composition is preferably 8% by mass or less, more preferably 6% by mass or less, and further preferably 4% by mass or less.

### <Ionic surfactant(s) other than component (A)>

From the viewpoint of improving sebum cleansing property and further improving foaming property, foam persistence and foam quality, the skin cleanser composition of the present invention may also comprise ionic surfactant(s) other than the above-mentioned component (A). Examples of ionic surfactant(s) other than the above-mentioned component (A) include anionic surfactant(s) other than the component (A), amphoteric surfactant(s), etc., preferably one or more selected from anionic surfactant(s) other than the component (A) and amphoteric surfactant(s).

Examples of the anionic surfactant(s) other than the component (A) include, an anionic surfactant having a sulfonic acid group or sulfate group, a phosphate ester anionic surfactant. Specifically, examples of the anionic surfactant having a sulfonic acid group or sulfate group include, alkylbenzene sulfonic acid, alkanesulfonic acid, alkenylsulfonic acid, alkyl sulfonic acid, acyl hydroxyethyl sulfonic acid, alkyl sulfate ester, alkyl ether sulfate ester, alkyl sulfosuccinic acid, sulfofatty acid methyl ester, fatty acid alkanolamide sulfate, monoacylglycerol sulfate. Examples of the phosphate ester anionic surfactant include alkyl phosphate salts, polyoxyethylene alkyl ether phosphate salts, alkylaryl ether phosphate salts, fatty acid amide ether phosphoric acids.

As amphoteric surfactants, examples include betaine-type surfactants. Specifically, as betaine-type surfactants, examples include carbonylbetaine, amidobetaine, amidosulfobetaine, sulfobetaine.

Although the ionic surfactant(s) other than the above-mentioned component (A) can further improve the foaming property, foam persistence and foam quality, they have an adverse effect on the detergency against pores, particularly detergency against keratotic plugs. Therefore, when the skin cleanser composition of the present invention comprises ionic surfactant(s) other than the component (A), the content of the anionic surfactant(s) other than the component (A) or the total content of the anionic surfactant(s) other than the component (A) and the amphoteric surfactant(s) in terms of acid is preferably less than 1.5% by mass, more preferably 1.4% by mass or less, and further preferably 1% by mass or less, and most preferably ionic surfactant(s) other than the component (A) are substantially not comprised.

The term "substantially not comprised" means that it is not intentionally added, and it may be comprised within a range that does not impair the effect of the present invention, for example, the content in the composition is 0.5% by mass or less, preferably 0.1% by mass or less, more preferably 0.05% by mass or less, and further preferably 0% by mass.

From the viewpoint of obtaining good detergency against pores and detergency against keratotic plugs while further improving foaming property, foam persistence and foam quality, the content of anionic surfactant(s) other than the component (A), or the total content of anionic surfactant(s) other than the component (A) and amphoteric surfactant(s), is greater than 0% by mass in terms of acid. In addition, from the same viewpoint, the mass ratio of the content of the anionic surfactant(s) other than the component (A) in terms of acid or the total content of the anionic surfactant(s) other than the component (A) and the amphoteric surfactant(s) in terms of acid to the content of the component (A) in terms of acid, (the content of anionic surfactant(s) other than the component (A) (in terms of acid) or the total content of anionic surfactant(s) other than the component (A) and amphoteric surfactant(s) (in terms of acid))/(the content of the component (A) (in terms of acid)), is greater than 0 and less than 1, preferably 0.9 or less, more preferably 0.8 or less, and further preferably 0.7 or less.

### <Component (B)>

The component (B) is a component having a keratotic plug-removing effect, and is one or more selected from 2-amino-2-hydroxymethyl-1,3-propanediol, 2-amino-2-methyl-1-propanol, 2-amino-2-methyl-1,3-propanediol, and monoethanolamine.

2-Amino-2-hydroxymethyl-1,3-propanediol is represented by the following structural formula (x1), and is a component also called "tris(hydroxymethyl)aminomethane" or "TRIS".

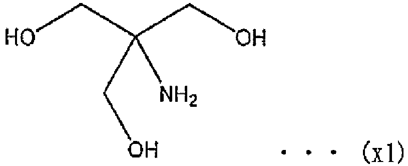

TRIS has a molecular weight of 121.14, a melting point of 169-173°C, and a boiling point of 219-220°C (10mmHg). In addition, it is a weakly alkaline component with a pKa of 8.03 at 25°C and is easily soluble in water.

2-Amino-2-methyl-1-propanol is a component represented by the following structural formula (x2), also referred to as "AMP".

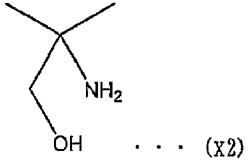

AMP has a molecular weight of 89.14, a melting point of 30-31°C, and a boiling point of 165.5°C (10 mmHg). In addition, it is a component that is easily soluble in water and has a pKa of 9.72 at 25°C.

2-Amino-2-methyl-1,3-propanediol is a component represented by the following structural formula (x3), and is also referred to as "AMPD".

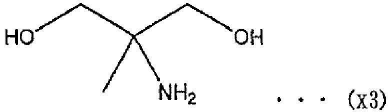

AMPD has a molecular weight of 105.14, a melting point of 107-112°C, and a boiling point of 151°C (10 mmHg). In addition, it is a component that is easily soluble in water and has a pKa of 8.76 at 25°C.

Monoethanolamine is a component represented by the following structural formula (x4), also referred to as "MEA".

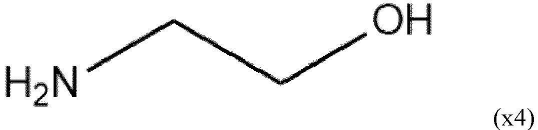

MEA has a molecular weight of 61.08, a melting point of 10-11°C, and a boiling point of 170.9°C (760 mmHg). In addition, it is a component that is easily soluble in water.

As the component (B), from the viewpoint of achieving even better detergency against pores and detergency against keratotic plugs, it is preferably one or more selected from 2-amino-2-hydroxymethyl-1,3-propanediol, 2-amino-2-methyl-1-propanol, 2-amino-2-methyl-1,3-propanediol, more preferably is 2-amino-2-hydroxymethyl-1,3-propanediol.

From the viewpoint of obtaining good detergency against keratotic plugs, the total content of the component (B) in the skin cleanser composition is preferably 0.08% by mass or more, more preferably 0.1% by mass or more, further preferably 0.3% by mass or more, further preferably 0.5% by mass or more, further preferably 0.8% by mass or more, further preferably 1% by mass or more, further preferably 2% by mass or more, further preferably 3% by mass or more, further preferably 4% by mass or more, further preferably 5% by mass or more. In addition, from the viewpoint of having excellent detergency against keratotic plugs and inhibiting the formation of crystals in the skin cleanser composition to prevent clogging of the foam discharge container, the total content of the component (B) in the skin cleanser composition is preferably 35% by mass or less, more preferably 30% by mass or less, further preferably 25% by mass or less, further preferably 20% by mass or less, further preferably 15% by mass or less, further preferably 12% by mass or less, further preferably 11% by mass or less, further preferably 10% by mass or less.

When the skin cleanser composition of the present invention comprises the component (A1), from the viewpoint of achieving both excellent detergency against pores, particularly detergency against keratotic plugs, and excellent foaming property, the mass ratio (B)/(A1) of the content of the component (B) to the content of the component (A1) in terms of acid is 0.5 or more, preferably 0.8 or more, more preferably 1.5 or more, further preferably 1.7 or more, further more preferably 3 or more, further more preferably 3.4 or more, and is 10 or less, preferably 7 or less, more preferably 5 or less, further preferably 4.5 or less.

When the skin cleanser composition of the present invention comprises the component (A2), from the viewpoint of achieving both excellent foaming property and excellent detergency against pores, particularly detergency against keratotic plugs, the mass ratio (B)/(A2) of the content of the component (B) to the content of the component (A2) in terms of acid is 0.3 or more, preferably 0.4 or more, more preferably 1 or more, and further preferably 6 or more, and is 43 or less, preferably 20 or less, more preferably 13 or less, further preferably 3.5 or less, and further more preferably 3.2 or less.

When the skin cleanser composition of the present invention comprises the component (A3), from the viewpoint of achieving both excellent foaming property and excellent detergency against pores, particularly detergency against keratotic plugs, the mass ratio (B)/(A3) of the content of the component (B) to the content of the component (A3) in terms of acid is preferably 1.5 or more, and is preferably 7 or less, and further preferably 6 or less.

### <Component (C)>

When the skin cleanser composition of the present invention is a foam discharge container-packed cleanser composition, the skin cleanser composition of the present invention preferably further comprises polyol(s) as component (C), and more preferably further comprises polyol(s) that is liquid at 25°C as component (C), from the viewpoint of inhibiting the formation of crystals in the skin cleanser composition, preventing clogging of the nozzle of the foam discharge container, and further improving the foam quality.

Examples of polyol(s) of the component (C) include di- to octavalent alcohols, with di- to hexavalent alcohols being preferred. As such polyols, examples include ethylene glycol, diethylene glycol, polyethylene glycol, 1,2-propylene glycol, 1,3-propylene glycol, dipropylene glycol, polypropylene glycol, 1,3-butylene glycol, isopentyldiol, glycerol, diglycerol, triglycerol, diethylene glycol monomethyl ether, diethylene glycol monoethyl ether, polyoxyalkylene glycerol ether, polyoxyalkylene diglycerol ether, polyoxyalkylene methyl glucoside, polyoxyalkylene ethyl glucoside, erythritol, xylitol, sorbitol. Wherein the polyoxyalkylene may be a homopolymer or a copolymer, and examples of such copolymers include polyoxyethylene-polyoxypropylene-polyoxybutylene copolymers and polyoxyethylene-polyoxypropylene copolymers.

**In** the skin cleanser composition of the present invention, the polyol may be used either individually or in combination of two or more.

From the viewpoint of further inhibiting the formation of crystals in the skin cleanser composition, preventing clogging of the nozzle of the foam discharge container, and further improving the foam quality, the component (C) is preferably one or more selected from 1,2-propylene glycol, 1,3-propylene glycol, dipropylene glycol, polypropylene glycol, 1,3-butylene glycol, glycerol, diethylene glycol monoethyl ether, polyoxyalkylene glycerol ether, polyoxyalkylene diglycerol ether, polyoxyalkylene methyl glucoside and sorbitol, more preferably one or more selected from 1,2-propylene glycol, 1,3-propylene glycol, dipropylene glycol, polypropylene glycol, glycerol, diethylene glycol monoethyl ether, polyoxyalkylene glycerol ether and polyoxyalkylene diglycerol ether. From the viewpoint of achieving both excellent detergency against pores, particularly detergency against keratotic plugs, and excellent foam quality, and further inhibiting the formation of crystals in the skin cleanser composition and preventing clogging of the foam discharge container, it is further preferably one or more selected from 1,2-propylene glycol, 1,3-propylene glycol, dipropylene glycol, and glycerol.

From the viewpoint of further inhibiting the formation of crystals in the skin cleanser composition, preventing clogging of the foam discharge container, and further improving the foam quality, the total content of the component (C) in the skin cleanser composition is preferably 3% by mass or more, more preferably 4% by mass or more, further preferably 7% by mass or more, and further more preferably 12.5% by mass or more, and is preferably 60% by mass or less, and more preferably 50% by mass or less. **In** addition, from the viewpoint of achieving both even better foam quality and detergency against pores, particularly detergency against keratotic plugs, it is preferably 35% by mass or less, more preferably 30% by mass or less, and further preferably 25% by mass or less.

From the viewpoint of further inhibiting the formation of crystals in the skin cleanser composition, preventing clogging of the foam discharge container, and further improving the foam quality, the mass ratio (C)/(B) of the content of the component (C) to the content of the component (B) is preferably 0.7 or more, more preferably 1.3 or more, further preferably 1.5 or more, and further more preferably 2.5 or more. Moreover, from the viewpoint of achieving even better detergency against keratotic plugs, particularly detergency against keratotic plugs, it is preferably 60 or less, more preferably 50 or less, and more preferably 45 or less.

**In** addition, from the viewpoint of inhibiting the formation of crystals in the skin cleanser composition, preventing clogging of the foam discharge container, and further improving the foam quality, the total content of the component (B) and the component (C) is preferably 10% by mass or more, more preferably 13% by mass or more, further preferably 16% by mass or more, further more preferably 18% by mass or more, and further more preferably 20% by mass or more.

### <Component (D)>

The skin cleanser composition of the present invention preferably further comprises nonionic surfactant(s) as component (D) from the viewpoint of further improving the foaming property.

As the component (D), from the viewpoint of further improving the foaming property, it is preferred to comprise nonionic surfactant(s) (D1) having an HLB of 11 or more. The HLB of the component (D1) is 11 or more, preferably 12 or more, more preferably 13 or more, and preferably 20 or less, more preferably 19 or less, and further preferably 18 or less. In addition, the HLB of the component (D1) is 11 or more, preferably 11 to 20, more preferably 12 to 19, and further preferably 13 to 18.

Herein, the HLB (Hydrophilic-Lypophilic Balance) of the nonionic surfactant, which represents the proportion of the molecular weight of the hydrophilic group portion in the total molecular weight of the nonionic surfactant, can be calculated by the following Griffin's formula. HLB = 20 × (molecular weight of the hydrophilic group portion in the surfactant molecule/molecular weight of the surfactant)

Examples of nonionic surfactant(s) of the component (D1) include, for example, polyglycerol fatty acid esters, polyethylene glycol fatty acid esters, polyoxyethylene glycerol fatty acid esters, polyoxyethylene polyoxypropylene glycol, polyoxyethylene sorbitan fatty acid esters, polyoxyethylene sorbitol fatty acid esters, polyoxyethylene castor oil, polyoxyethylene hydrogenated castor oil, polyoxyethylene hydrogenated castor oil fatty acid esters, polyoxyethylene alkyl ethers, polyoxyethylene alkyl ether fatty acid esters, sucrose fatty acid esters, alkyl polyglucosides, (poly)alkyl glyceryl ethers.

Among them, from the viewpoint of improving sebum cleansing ability, rinsing performance and foaming property, it is preferred to comprise one or more selected from polyglycerol fatty acid esters, polyethylene glycol fatty acid esters, polyoxyethylene glycerol fatty acid esters, polyoxyethylene sorbitan fatty acid esters, polyoxyethylene hydrogenated castor oil fatty acid esters, polyoxyethylene alkyl ethers and alkyl polyglucosides, and more preferably one or more selected from polyethylene glycol fatty acid esters, polyoxyethylene alkyl ethers, polyoxyethylene sorbitan fatty acid esters and alkyl polyglucosides.

As the component (D), from the viewpoint of exhibiting better makeup removal property, it is preferred to comprise nonionic surfactant(s) (D2) having an HLB of less than 11. The HLB of component (D2) is less than 11, preferably 10 or less, more preferably 9 or less, and is preferably 4 or more, more preferably HLB 5 or more, and more preferably HLB 6 or more. In addition, the HLB of the component (D2) is less than 11, preferably 4 to 10, more preferably 5 to 10, and further preferably 6 to 9.

Examples of nonionic surfactant(s) of the component (D2) include polyethylene glycol-based surfactants such as ethylene glycol fatty acid esters such as ethylene glycol monostearate, polyethylene glycol fatty acid esters such as polyethylene glycol (2) monostearate, polyethylene glycol alkyl ethers such as polyethylene glycol (5) decyl pentadecyl ether, polyethylene glycol hydrogenated castor oil such as polyethylene glycol (5) hydrogenated castor oil monoisolaurate; propylene glycol-based surfactants such as propylene glycol fatty acid esters, polypropylene glycol fatty acid esters, propylene glycol alkyl ethers, polypropylene glycol alkyl ethers, and ethylene oxide derivatives of propylene glycol alkyl ethers; glycerol fatty acid esters such as glycerol monoisostearate; glycerol alkyl ethers such as glycerol monoisostearyl ether; sorbitan fatty acid esters such as sorbitan monostearate; polyglycerol fatty acid esters such as polyglycerol monoisostearate, etc.

Among them, it is preferred to comprise one or more selected from polyoxyethylene alkyl ethers and polyglycerol fatty acid esters.

By comprising nonionic surfactant(s) in the skin cleanser composition of the present invention, the foaming property can be further improved. However, if the content of nonionic surfactant(s) is too high, it will have an adverse effect on the detergency against pores. Therefore, the total content of the component (D) is preferably 10% by mass or less, and more preferably 8% by mass or less.

### <Other components>

The skin cleanser composition of the present invention may further comprise components commonly used in skin cleanser compositions, in addition to the above-mentioned components, such as oils such as hydrocarbon oils, ester oils, ether oils, and vegetable oils; moisturizing agents such as sodium lactate, urea, and sodium pyrrolidone carboxylate; pH regulators such as sodium hydroxide, potassium hydroxide, sodium carbonate, sodium citrate, hydrochloric acid, and malic acid; antioxidants; bactericides; extracts; fragrances; pigments; and the like, within a range that does not impair the effects of the present invention.

From the viewpoint of improving detergency against pores, particularly detergency against keratotic plugs, the pH value of the skin cleanser composition of the present invention at 25°C is preferably 8.5 or more, more preferably 8.8 or more, further preferably 9 or more, further preferably 9.2 or more, further preferably 9.3 or more, further preferably 9.5 or more, further preferably 9.8 or more, and further preferably 10 or more. In addition, from the viewpoint of inhibiting excessive burden on the skin, the pH value of the skin cleanser composition of the present invention at 25°C is preferably 12.5 or less, more preferably 12.2 or less, further preferably 12 or less, further preferably 11.8 or less, further preferably 11.5 or less, further preferably 11.2 or less, further preferably 11 or less, further preferably 10.5 or less.

The dosage form of the skin cleanser composition of the present invention is not particularly limited, and is preferably a foam, liquid, paste, cream, etc. From the viewpoint of being easy to use and improving penetration into keratotic plugs in pores, it is more preferably a foam or liquid form. The skin cleanser composition of the present invention can be suitably used as facial cleanser, makeup cleansing cosmetic, body cleanser, etc.

When the skin cleanser composition of the present invention is packed in a foam discharge container for application in a foam dosage form, there is no particular limitation on the foam discharge container containing the skin cleanser composition of the present invention, as long as a foam discharge container commonly used in the art is used. Furthermore, from the viewpoint of obtaining good foam quality, a gas-liquid mixing ratio is 10/1 or more, preferably 12/1 or more, more preferably 13/1 or more, more preferably 16/1 or more, further preferably 17/1 or more, and further more preferably 20/1 or more, in terms of the volume ratio of air to the cleanser composition (air/cleanser composition). Furthermore, from the viewpoint of miniaturization of the foam discharge container, the gas-liquid mixing ratio is 38/1 or less, preferably 30/1 or less, further preferably 29/1 or less, and further more preferably 26/1 or less.

The skin cleanser composition of the present invention can be obtained, for example, by a manufacturing method having the steps of sequentially adding the component (A), the component (B), and, if necessary, the component (C), the component (D), and other components to water, and mixing and stirring. Additionally, if necessary, water can be preheated to 60-80°C, and then components such as the component (A), the component (B) can be sequentially added to the water and mixed and stirred. Subsequently, the resulting mixture can be cooled to 20-35°C.

The method of using the skin cleanser composition of the present invention can be exemplified by applying the skin cleanser composition of the present invention to the skin of the body, preferably to the skin of face, neck, hands, feet, or torso excluding the scalp, more preferably to the pores areas of these skin regions, and further preferably to the pores areas of the skin from the forehead to the tip of the nose, typically leaving it on for 10 seconds to 30 minutes, followed by rinsing off the skin cleanser composition with water or wiping off the skin cleanser composition with a wipe material such as a paper towel, nonwoven fabric, or fabric, preferably rinsing off with water. In addition, after applying the skin cleanser composition of the present invention, massage may be omitted or performed on the applied area for 10 seconds to 10 minutes, followed by rinsing off the skin cleanser composition with water or wiping off the skin cleanser composition with a wipe material such as a paper towel, nonwoven fabric, or fabric, preferably rinsing off with water. In addition, the skin cleanser composition of the present invention can be applied to the pores of the skin by using hands or tools such as nonwoven fabric, fabric, and brush.

Regarding the above embodiments, the present invention further discloses the following skin cleanser composition.
<1> A skin cleanser composition comprising the following component (A) and component (B),
   component (A) is one or more anionic surfactant(s) selected from the following components (A1), (A2), and (A3),
   component (A1) is an N-acyl amino acid represented by the following general formula (1), or a salt thereof, 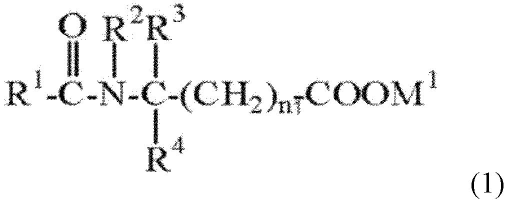
   (In formula (1), R¹ represents a linear or branched alkyl or alkenyl with 5 to 24 carbon atoms. R² represents hydrogen or an alkyl with 1 to 4 carbon atoms. R³ and R⁴ are the same or different and represent hydrogen, an alkyl with 1 to 4 carbon atoms, a hydroxyalkyl with 1 to 4 carbon atoms, or -(CH₂)ₘ₁-COOM². m1 and n1 are each independently a number from 0 to 20. M¹ and M² are the same or different and represent hydrogen, an alkali metal, an alkaline earth metal, a basic amino acid, ammonium or alkanolamine.)
   component (A2) is an N-acyl taurine represented by the following general formula (2), or a salt thereof,

      R₁CO-NR₂-CH₂CH₂SO₃M (2)
   (In formula (2), R¹ represents a linear or branched alkyl or alkenyl with 8 to 20 carbon atoms. R₂ represents hydrogen or an alkyl with 1 to 3 carbon atoms. M represents hydrogen, an alkali metal, an alkaline earth metal, a basic amino acid, ammonium, or alkanolamine.)
   component (A3) is a fatty acid with 8 to 20 carbon atoms, or a salt thereof;
   component (B) is one or more selected from 2-amino-2-hydroxymethyl-1,3-propanediol, 2-amino-2-methyl-1- propanol, 2-amino-2-methyl-1,3-propanediol and monoethanolamine.
<2> The skin cleanser composition according to above <1>, wherein when the component (A) comprises the component (A1), the content of the component (A1) in terms of acid is 0.5-15% by mass, and the mass ratio (B)/(A1) of the content of the component (B) to the content of the component (A1) in terms of acid is 0.5 to 10,
   when the component (A) comprises the component (A2), the content of the component (A2) in terms of acid is 0.1-25% by mass, and the mass ratio (B)/(A2) of the content of the component (B) to the content of the component (A2) in terms of acid is 0.3 to 43,
   when the component (A) comprises the component (A3), the content of the component (A3) in terms of acid is 1-8% by mass, and the mass ratio (B)/(A3) of the content of the component (B) to the content of the component (A3) in terms of acid is 1.5 to 7,
   ionic surfactant(s) other than the component (A) may or may not be comprised, wherein the content of anionic surfactant(s) other than the component (A) or the total content of anionic surfactant(s) other than the component (A) and amphoteric surfactant(s) in terms of acid is less than 1.5% by mass.
<3> The skin cleanser composition according to above <1> or <2>, wherein in formula (1), R³ and R⁴ are each independently an alkyl with 1 to 4 carbon atoms, a hydroxyalkyl with 1 to 4 carbon atoms, or -(CH₂)ₘ₁-COOM², preferably an alkyl with 1 to 4 carbon atoms, or -(CH₂)ₘ₁-COOM^{?}, more preferably -(CH₂)ₘ₁-COOM².
<4> The skin cleanser composition according to any one of above <1> to <3>, wherein the component (A1) is one or more selected from N-acyl glycine or a salt thereof, N-acyl alanine or a salt thereof, N-acyl threonine or a salt thereof, N-acyl serine or a salt thereof, N-acyl sarcosine or a salt thereof, N-acyl glutamic acid or a salt thereof, and N-acyl aspartic acid or a salt thereof, preferably one or more selected from N-acyl amino acids with a branched structure or salts thereof, more preferably one or more selected from N-acyl alanine or a salt thereof, N-acyl glutamic acid or a salt thereof, N-acyl threonine or a salt thereof, N-acyl serine or a salt thereof, and N-acyl aspartic acid or a salt thereof, further preferably one or more selected from N-acyl glutamic acid or a salt thereof, N-acyl aspartic acid or a salt thereof.
<5> The skin cleanser composition according to any one of above <1> to <4>, wherein when the skin cleanser composition comprises the component (A1), the content of the component (A1) in terms of acid is 0.5% by mass or more, preferably 0.8% by mass or more, more preferably 1% by mass or more, and is 15% by mass or less, preferably 7.5% by mass or less, more preferably 5% by mass or less, and further preferably 4% by mass or less.
<6> The skin cleanser composition according to any one of above <1> to <5>, wherein when the skin cleanser composition comprises the component (A1), the mass ratio (B)/(A1) of the content of the component (B) to the content of the component (A1) in terms of acid is 0.5 or more, preferably 0.8 or more, more preferably 1.5 or more, further preferably 1.7 or more, further more preferably 3 or more, further more preferably 3.4 or more, and is 10 or less, preferably 7 or less, more preferably 5 or less, further preferably 4.5 or less.
<7> The skin cleanser composition according to any one of above <1> to <6>, wherein the component (A2) is one or more selected from N-lauroyl-N-methyltaurine or a salt thereof, N-myristoyl-N-methyltaurine or a salt thereof, N-cocoyl-N-methyltaurine or a salt thereof, N-lauroyl-N-ethyltaurine or a salt thereof, N-myristoyl-N-ethyltaurine or a salt thereof, N-cocoyl-N-ethyltaurine or a salt thereof, N-lauroyltaurine or a salt thereof, and N-cocoyltaurine or a salt thereof.
<8> The skin cleanser composition according to any one of above <1> to <7>, wherein when the skin cleanser composition comprises the component (A2), the content of the component (A2) in terms of acid is 0.1% by mass or more, preferably 0.4% by mass or more, more preferably 1.5% by mass or more, and further preferably 1.8% by mass or more, and is 25% by mass or less, preferably 23% by mass or less, more preferably 15% by mass or less, and further preferably 6% by mass or less.
<9> The skin cleanser composition according to any one of above <1> to <8>, wherein when the skin cleanser composition comprises the component (A2), the mass ratio (B)/(A2) of the content of the component (B) to the content of the component (A2) in terms of acid is 0.3 or more, preferably 0.4 or more, more preferably 1 or more, and further preferably 6 or more, and is 43 or less, preferably 20 or less, more preferably 13 or less, further preferably 3.5 or less, and further preferably 3.2 or less.
<10> The skin cleanser composition according to any one of above <1> to <9>, wherein the component (A3) is one or more selected from lauric acid, myristic acid, palmitic acid, stearic acid, behenic acid, and salts thereof.
<11> The skin cleanser composition according to any one of above <1> to <10>, wherein when the skin cleanser composition comprises the component (A3), the content of the component (A3) in terms of acid is 1% by mass or more, preferably 2% by mass or more, and is 8% by mass or less, preferably 6% by mass or less, and more preferably 4% by mass or less.
<12> The skin cleanser composition according to any one of above <1> to <11>, wherein when the skin cleanser composition comprises the component (A3), the mass ratio (B)/(A3) of the content of the component (B) to the content of the component (A3) in terms of acid is 1.5 or more, and is 7 or less, preferably 6 or less.
<13> The skin cleanser composition according to any one of above <1> to <12>, wherein the content of anionic surfactant(s) other than the component (A) , or the total content of anionic surfactant(s) other than the component (A) and amphoteric surfactant(s), is preferably 1.4% by mass or less, further preferably 1% by mass or less in terms of acid, and most preferably ionic surfactant(s) other than the component (A) are substantially not comprised.
<14> The skin cleanser composition according to any one of above <1> to <13>, wherein the content of anionic surfactant(s) other than the component (A), or the total content of anionic surfactant(s) other than the component (A) and amphoteric surfactant(s), is greater than 0% by mass in terms of acid,
   the mass ratio of the content of the anionic surfactant(s) other than the component (A) in terms of acid or the total content of the anionic surfactant(s) other than the component (A) and the amphoteric surfactant(s) in terms of acid to the content of the component (A) in terms of acid, (the content of anionic surfactant(s) other than the component (A) (in terms of acid) or the total content of anionic surfactant(s) other than the component (A) and amphoteric surfactant(s) (in terms of acid))/(the content of the component (A) (in terms of acid)), is greater than 0 and less than 1, preferably 0.9 or less, more preferably 0.8 or less, and further preferably 0.7 or less.
<15> The skin cleanser composition according to any one of above <1> to <14>, wherein the component (B) is one or more selected from 2-amino-2-hydroxymethyl-1,3-propanediol, 2-amino-2-methyl-1-propanol, and 2-amino-2-methyl-1,3-propanediol, and preferably 2-amino-2-hydroxymethyl-1,3-propanediol.
<16> The skin cleanser composition according to any one of above <1> to <15>, wherein the total content of the component (B) in the skin cleanser composition is 0.08% by mass or more, preferably 0.1% by mass or more, more preferably 0.3% by mass or more, further preferably 0.5% by mass or more, further preferably 0.8% by mass or more, further preferably 1% by mass or more, further preferably 2% by mass or more, further preferably 3% by mass or more, further preferably 4% by mass or more, further preferably 5% by mass or more, and is 35% by mass or less, preferably 30% by mass or less, more preferably 25% by mass or less, further preferably 20% by mass or less, further preferably 15% by mass or less, further preferably 12% by mass or less, further preferably 11% by mass or less, further preferably 10% by mass or less.
<17> The skin cleanser composition according to any one of above <1> to <16>, wherein when the skin cleanser composition is a foam discharge container-packed skin cleanser composition, the skin cleanser composition further comprises (C) polyol(s).
<18> The skin cleanser composition according to above <17>, wherein the component (C) is polyol(s) which is liquid at 25°C, and is preferably one or more selected from 1,2-propylene glycol, 1,3-propylene glycol, dipropylene glycol, polypropylene glycol, 1,3-butylene glycol, glycerol, diethylene glycol monoethyl ether, polyoxyalkylene glycerol ether, polyoxyalkylene diglycerol ether, polyoxyalkylene methyl glucoside, and sorbitol; more preferably one or more selected from 1,2-propylene glycol, 1,3-propylene glycol, dipropylene glycol, polypropylene glycol, glycerol, diethylene glycol monoethyl ether, polyoxyalkylene glycerol ether, and polyoxyalkylene diglycerol ether; and further preferably one or more selected from 1,2-propylene glycol, 1,3-propylene glycol, dipropylene glycol, and glycerol.
<19> The skin cleanser composition according to above <17> or <18>, wherein the total content of the component (C) is 3% by mass or more, preferably 4% by mass or more, more preferably 7% by mass or more, further preferably 12.5% by mass or more, and is 60% by mass or less, preferably 50% by mass or less, more preferably 35% by mass or less, further preferably 30% by mass or less, and further more preferably 25% by mass or less.
<20> The skin cleanser composition according to any one of above <17> to <19>, wherein the mass ratio (C)/(B) of the content of the component (C) to the content of the component (B) is 0.7 or more, more preferably 1.3 or more, further preferably 1.5 or more, further preferably 2.5 or more, and is 60 or less, preferably 50 or less, and further preferably 45 or less.
<21> The skin cleanser composition according to any one of above <17> to <20>, wherein the total content of the component (B) and the component (C) is 10% by mass or more, preferably 13% by mass or more, more preferably 16% by mass or more, further preferably 18% by mass or more, and further preferably 20% by mass or more.
<22> The skin cleanser composition according to any one of above <1> to <21>, further comprising a nonionic surfactant as component (D).
<23> The skin cleanser composition according to above <22>, wherein the component (D) comprises nonionic surfactant(s) having an HLB of 11 or more as component (D1).
<24> The skin cleanser composition according to above <23>, wherein the component (D1) comprises one or more selected from polyglycerol fatty acid esters, polyethylene glycol fatty acid esters, polyoxyethylene glycerol fatty acid esters, polyoxyethylene sorbitan fatty acid esters, polyoxyethylene hydrogenated castor oil fatty acid esters, polyoxyethylene alkyl ethers and alkyl polyglucosides, preferably one or more selected from polyethylene glycol fatty acid esters, polyoxyethylene alkyl ethers, polyoxyethylene sorbitan fatty acid esters and alkyl polyglucosides.
<25> The skin cleanser composition according to above <22>, wherein the component (D) comprises nonionic surfactant(s) having an HLB of less than 11 as component (D2).
<26> The skin cleanser composition as according to above <25>, wherein the component (D2) comprises one or more selected from polyoxyethylene alkyl ethers and polyglycerol fatty acid esters.
<27> The skin cleanser composition according to any one of above <22> to <26>, wherein the total content of the component (D) is 10% by mass or less, preferably 8% by mass or less.
<28> The skin cleanser composition according to any one of above <1> to <27>, wherein the pH value of the skin cleanser composition at 25°C is 8.5 or more, preferably 8.8 or more, more preferably 9 or more, further preferably 9.2 or more, further preferably 9.3 or more, further preferably 9.5 or more, further preferably 9.8 or more, further preferably 10 or more, and is 12.5 or less, preferably 12.2 or less, more preferably 12 or less, further preferably 11.8 or less, further preferably 11.5 or less, further preferably 11.2 or less, further preferably 11 or less, and further more preferably 10.5 or less.
<29> A method for removing keratotic plugs, wherein the skin cleanser composition according to any one of above <1> to <28> is applied to the skin of the body, preferably to the skin of face, neck, hands, feet, or torso excluding the scalp, more preferably to the pores areas of these skin regions, and further preferably to the pores areas of the skin from the forehead to the tip of the nose, and after a certain period of time, the composition is rinsed off with water or wiped off with a wiping material.

### EXAMPLES

Hereinafter, the invention will be described in more detail by way of examples. The examples are merely illustrative of the present invention, and are not intended to be limiting in any way. Using the components shown in the following tables, the skin cleanser compositions of the examples and comparative examples were obtained. Unless otherwise indicated in the tables, the content of each component is represented by mass %.

### Examples 1-32 and Comparative Example 1

The samples were prepared according to the formulations shown in Tables 1 to 4. Specifically, when the component (A1) was used, the remaining components were added sequentially to water, mixed and stirred, to obtain the sample; when the component (A2) and/or the component (A3) were used, water was preheated to 70°C, the remaining components were added sequentially, mixed and stirred, and the resulting mixture was cooled to 20°C to obtain the sample.

In addition, the pH value of the obtained sample was measured at 25°C by using a pH meter (manufactured by Horiba, Ltd., Model No. F-22). Furthermore, detergency against pores and foaming property of the obtained samples were evaluated by the following method. The obtained results were shown in Tables 1 to 4.

### <Evaluation of detergency against pores>

"Biore Nose Pack (manufactured by Kao Corporation)" was used to remove the keratotic plugs from the nose, and the collected keratotic plugs were gently taken out with a tweezer.

The collected keratotic plugs were placed on a slide glass (MATSUNAMI Glass Co., Ltd., 76×26mm), covered with a cover glass, and 0.05mL of the sample was dropwise added to the edge of the cover glass. Thus, the sample could enter the gap between the slide glass and the cover glass through capillary phenomenon, and is brought into contact with the keratotic plugs. In addition, the contact between the keratotic plugs and the sample was recorded with a digital microscope (VHX-5000; manufactured by Keyence Corporation, magnification 150 times), and the collapse state of the keratotic plugs was evaluated after 1 minute from the contact of the keratotic plugs with the sample and then rinsing with deionized water. Each evaluation result was recorded in the tables as "detergency against pores". In addition, the measurement was performed at 25°C.

The detergency against pores was relatively evaluated by 6 criteria from the state 1 "almost no change is observed in the appearance of the keratotic plugs" to the state 6 "peeling and separation is observed from surface layer to the central of the keratotic plugs (initial appearance of the keratotic plugs is not retained)".

### <Evaluation of foaming property>

Four expert panelists dropwise applied 1 mL of the sample onto their previously wetted hands, rubbed the palms of both hands together for 10 seconds to foam the sample. The state of the foam at this time was evaluated according to the following criteria. The evaluation results were presented as the average value of the results evaluated by the four expert panelists.

### (Criteria)

5: More foam
4: Slightly more foam
3: Some degree of foam
2: Almost no foam
1: Not foamed at all
Note: The components used in the examples and comparative examples were shown below.

### <Component (A)>

<Component (A1)>
(A1-1): Sodium cocoyl glutamate (trade name: AMISOFT CS-22B, manufacturer: Ajinomoto Co., Ltd., containing 20% of disodium cocoyl glutamate and 5% of monosodium cocoyl glutamate)
(A1-2): Sodium N-lauroyl-N-methyl-β-alanine (trade name: ALANON ALE, manufacturer: Kawaken Fine Chemicals Co., Ltd.)
(A1-3): Potassium cocoyl glycinate (trade name: AMILITE GCK-12K, manufacturer: Ajinomoto Co., Ltd., containing 30% of potassium cocoyl glycinate)
(A1-4): Sodium N-lauroyl-L-aspartate (trade name: AMINOFOAMER FLDS-LK, manufacturer: ASAHI KASEI FINECHEM CO., LTD.)

<Component (A2)>
(A2-1): Sodium N-lauroyl-N-methyltaurine (trade name: NIKKOL LMT, manufacturer: NOF CO., LTD.)
<Component (A3)>
(A3-1): Lauric acid (trade name: LAURIC ACID, manufacturer: Acidchem International SDN. BHD.)
<Component (A')> [Ionic surfactants other than component (A)]
(A'-1): Polyoxyethylene (5) lauryl ether carboxylic acid (trade name: KAO AKYPO RLM-45, manufacturer: Kao Corporation) (containing 90% of polyoxyethylene (5) lauryl ether carboxylic acid)
(A'-2): Sodium polyoxyethylene (2) alkyl (C10-16) ether sulfate (trade name: Emal 227-PH11K, manufacturer: Kao Corporation)
(A'-3): Mixture of sodium hexadecyl hydroxysulfonate and sodium hexadecene sulfonate (trade name: PELEX DH-60, manufacturer: Kao Corporation)

<Component (B)>
(B-1): 2-amino-2-hydroxymethyl-1,3-propanediol (trade name: TRIS AMINO ULTRA PC (J) TROMETHAMINE, manufacturer: ANGUS Chemical Company)
(B-2): 2-amino-2-methyl-1,3-propanediol (trade name: AMPD, manufacturer: Tokyo Chemical Industry Co., Ltd.)
(B-3): 2-amino-2-methyl-1-propanol (trade name: AMP, manufacturer: Damas-beta)
(B-4): Monoethanolamine (trade name: Monoethanolamine, manufacturer: Damas-beta)

<Component (C)>
(C-1): 1,2-propanediol (trade name: PROPYLENE GLYCOL (COSMETIC GRADE), manufacturer: ADEKA Corporation)
(C-2): Polyoxypropylene diglyceryl ether (trade name: SY-DP9, manufacturer: Sakamoto Yakuhin Kogyo Co., Ltd.)
(C-3): Diethylene glycol monoethyl ether (trade name: CARBITOL SOLVENT, manufacturer: Dow Chemical Company)
(C-4): Dipropylene glycol (trade name: DPG-RF, manufacturer: ADEKA Corporation)
(C-5): Polyoxyethylene (10) methyl glucoside (trade name: GLUCAM E-10, manufacturer: The Lubrizol Corporation)
(C-6): 1,3-butylene glycol (trade name: 1,3-BUTYLENE GLYCOL-P, manufacturer: KH Neochem Co., Ltd.)
(C-7): 1,3-propylene glycol (trade name: ZEMEA SELECT PROPANEDIOL, manufacturer: DuPont)
(C-8): Glycerin (trade name: GLYCERIN A (COSMETIC GRADE), manufacturer: Kao Corporation)
(C-9): D-sorbitol (trade name: SORBITOL #650, manufacturer: Mitsubishi Corporation Life Sciences Limited)
(C-10): Polypropylene glycol (7) (trade name: ADEKA CARPOL DL-30, manufacturer: ADEKA Corporation, Mw = 400)
(C-11): Polyoxyethylene (8)/polyoxypropylene (5)/polyoxybutylene (3) copolymer glyceryl ether (trade name: WILBRIDE S-753, manufacturer: NOF Corporation)

<Component (D)>
(D-1): Oligomeric D-glucopyranose C9-11-alkyl glycoside (trade name: AG-10LK, manufacturer: Kao Corporation) (containing 40% of oligomeric D-glucopyranose C9-11-alkyl glycoside)

**[Table 1]**

| | Example 1 | Example 2 | Example 3 | Example 4 | Example 5 | Example 6 | Example 7 | Example 8 | Example 9 | Example 10 | Comparative Example 1 |
|---|---|---|---|---|---|---|---|---|---|---|---|
| (A1-1) | 6 | 8 | 12 | 16 | 32 | | | | | 6 | |
| (A1-2) | | | | | | 6 | 8 | | | | |
| (A1-3) | | | | | | | | 6 | 8 | | |
| (A'-1) | | | | | | | | | | 1.11 | 12 |
| (B-1) | 6 | 6 | 6 | 6 | 6 | 6 | 6 | 6 | 6 | 6 | 6 |
| pH regulator*¹ | q.s. | q.s. | q.s. | q.s. | q.s. | q.s. | q.s. | q.s. | q.s. | q.s. | q.s. |
| Water | Balance | Balance | Balance | Balance | Balance | Balance | Balance | Balance | Balance | Balance | Balance |
| Total | 100 | 100 | 100 | 100 | 100 | 100 | 100 | 100 | 100 | 100 | 100 |
| pH | 10.05 | 10.06 | 10.03 | 10.01 | 9.98 | 10.03 | 10.03 | 10.05 | 10.05 | 10.04 | 10.01 |
| The content of component (A) | 1.5 | 2.0 | 3.0 | 4.0 | 8.0 | 1.8 | 2.4 | 1.8 | 2.4 | 1.5 | 0 |
| The content of component (A) in terms of acid | 1.34 | 1.79 | 2.68 | 3.57 | 7.14 | 1.67 | 2.23 | 1.57 | 2.09 | 1.34 | 0 |
| The content of ionic surfactant(s) other than component (A) in terms of acid | 0 | 0 | 0 | 0 | 0 | 0 | 0 | 0 | 0 | 1.0 | 10.8 |
| The content of component (B) | 6 | 6 | 6 | 6 | 6 | 6 | 6 | 6 | 6 | 6 | 6 |
| Component (B)/Component (A)(in terms of acid) | 4.5 | 3.4 | 2.2 | 1.7 | 0.8 | 3.6 | 2.7 | 3.8 | 2.9 | 4.5 | - |
| Detergency against pores | 5 | 5 | 4 | 4 | 3 | 4 | 4 | 4 | 3 | 3 | 2 |
| Foaming Property | 3.0 | 3.0 | 4.0 | 4.0 | 5.0 | 4.0 | 4.0 | 4.0 | 5.0 | 3.0 | 3.0 |

| | | | | | | | | | | | |
|---|---|---|---|---|---|---|---|---|---|---|---|
| * 1: 0.5M of hydrochloric acid or 48% of potassium hydroxide was used as a pH regulator | | | | | | | | | | | |

**[Table 2]**

| | Example 11 | Example 12 | Example 13 | Example 14 | Example 15 | Example 16 | Example 17 | Example 18 | Example 19 | Example 20 | Example 21 | Example 22 |
|---|---|---|---|---|---|---|---|---|---|---|---|---|
| (A2-1) | 0.5 | 1 | 2 | 4 | 6 | 8 | 12 | 16 | 24 | | | |
| (A3-1) | | | | | | | | | | 1 | 2 | 4 |
| (B-1) | 6 | 6 | 6 | 6 | 6 | 6 | 6 | 6 | 6 | 6 | 6 | 6 |
| pH regulator*¹ | q.s. | q.s. | q.s. | q.s. | q.s. | q.s. | q.s. | q. s. | q.s. | q.s. | q.s. | q.s. |
| Water | Balance | Balance | Balance | Balance | Balance | Balance | Balance | Balance | Balance | Balance | Balance | Balance |
| Total | 100 | 100 | 100 | 100 | 100 | 100 | 100 | 100 | 100 | 100 | 100 | 100 |
| pH | 10.04 | 10.05 | 10.04 | 10.03 | 10.01 | 10.00 | 10.00 | 9.97 | 9.96 | 10.02 | 10.02 | 10.00 |
| The content of component (A) | 0.5 | 1.0 | 2.0 | 4.0 | 6.0 | 8.0 | 12.0 | 16.0 | 24.0 | 1.0 | 2.0 | 4.0 |
| The content of component (A) in terms of acid | 0.47 | 0.94 | 1.87 | 3.74 | 5.62 | 7.49 | 11.23 | 14.98 | 22.47 | 1.0 | 2.0 | 4.0 |
| The content of component (B) | 6 | 6 | 6 | 6 | 6 | 6 | 6 | 6 | 6 | 6 | 6 | 6 |
| Component (B)/Component (A)(in terms of acid) | 12.8 | 6.4 | 3.2 | 1.6 | 1.1 | 0.8 | 0.5 | 0.4 | 0.3 | 6.0 | 3.0 | 1.5 |
| Detergency against pores | 6 | 6 | 5 | 5 | 5 | 4 | 4 | 4 | 3 | 4 | 4 | 4 |
| Foaming Property | 3.0 | 3.0 | 4.0 | 4.0 | 4.0 | 5.0 | 5.0 | 6.0 | 6.0 | 3.0 | 5.0 | 5.0 |

**[Table 3]**

| | Example 23 | Example 24 | Example 25 | Example 26 | Example 27 | Example 28 |
|---|---|---|---|---|---|---|
| (A1-1) | 6 | 6 | 6 | | | |
| (A2-1) | | | | 6 | 6 | 6 |
| (B-2) | 6 | | | 6 | | |
| (B-3) | | 6 | | | 6 | |
| (B-4) | | | 6 | | | 6 |
| pH regulator*¹ | q.s. | q.s. | q.s. | q.s. | q.s. | q.s. |
| Water | Balance | Balance | Balance | Balance | Balance | Balance |
| Total | 100 | 100 | 100 | 100 | 100 | 100 |
| pH | 10.1 | 10.3 | 10.3 | 10.0 | 10.2 | 10.3 |
| The content of component (A) | 1.5 | 1.5 | 1.5 | 6.0 | 6.0 | 6.0 |
| The content of component (A) in terms of acid | 1.34 | 1.34 | 1.34 | 5.62 | 5.62 | 5.62 |
| The content of component (B) | 6 | 6 | 6 | 6 | 6 | 6 |
| Component (B)/Component (A)(in terms of acid) | 4.5 | 4.5 | 4.5 | 1.1 | 1.1 | 1.1 |
| Detergency against pores | 5 | 4 | 3 | 5 | 5 | 4 |
| Foaming Property | 3.0 | 3.0 | 3.0 | 4.0 | 4.0 | 4.0 |

**[Table 4]**

| | Example 29 | Example 30 | Example 31 | Example 32 |
|---|---|---|---|---|
| (A1-1) | 6 | 6 | 6 | 6 |
| (B-1) | 6 | 6 | 6 | 6 |
| (D-1) | 2.5 | 3.8 | 20 | 25 |
| pH regulator*¹ | q.s. | q.s. | q.s. | q.s. |
| Water | Balance | Balance | Balance | Balance |
| Total | 100 | 100 | 100 | 100 |
| pH | 10.05 | 10.00 | 9.98 | 10.01 |
| The content of component (A) | 1.5 | 1.5 | 1.5 | 1.5 |
| The content of component (A) in terms of acid | 1.34 | 1.34 | 1.34 | 1.34 |
| The content of component (B) | 6 | 6 | 6 | 6 |
| Component (B)/Component (A)(in terms of acid) | 4.5 | 4.5 | 4.5 | 4.5 |
| The content of component (D) | 1.0 | 1.5 | 8.0 | 10.0 |
| Detergency against pores | 5 | 5 | 4 | 3 |
| Foaming Property | 4.0 | 5.0 | 6.0 | 6.0 |

As can be seen from above Tables 1 to 4, the skin cleanser compositions obtained in the examples can achieve both excellent detergency against pores and excellent foaming property.

On the other hand, according to Comparative Example 1, it can be seen that when the specific anionic surfactant of the present invention is not used, it is impossible to achieve both detergency against pores and foaming property.

### Examples 33 to 75

The samples were prepared according to the formulations shown in Tables 5 to 10. Specifically, when the component (A1) was used, the remaining components were added sequentially to water, mixed and stirred, to obtain the sample; when the component (A2) and/or the component (A3) were used, water was preheated to 70°C, the remaining components were added sequentially, mixed and stirred, and the resulting mixture was cooled to 20°C to obtain the sample. Then, each of the obtained samples was filled into the foam discharge container (manufacturer: Yoshino Kogyo Co., Ltd., a pump-shaped device equipped with a gas-liquid mixing mechanism described in JP-A-2019-107644, gas-liquid mixing ratio 20/1), thereby preparing the foam discharge container-packed skin cleanser compositions of Examples 33 to 75.

Furthermore, the pH values of the obtained compositions were measured at 25°C by using a pH meter (manufactured by Horiba, Ltd., Model No. F-22). The results showed that the pH values of the foam discharge container-packed skin cleanser compositions of Examples 33 to 75 were all within the range of 9.8 to 10.5.

In addition, detergency against pores of the obtained samples was evaluated by the above method, and the foam quality of the foam discharged from the foam discharge container and the possibility of nozzle clogging of the foam discharge container were also evaluated by the following methods. The obtained results were shown in Tables 5 to 10.

### <Foam quality of foam discharged from the foam discharge container>

The foam was discharged from the foam discharge container onto a previously wetted hand (liquid volume 4 mL), the foam was immediately pressed with the other hand from above the discharged foam, and then the foam quality of the foam felt with the other hand was evaluated based on the following criteria. The evaluation results were presented as the average value of the evaluations of 4 expert panelists.
5: Very good foam quality.
4: Good foam quality.
3: Slightly good foam quality.
2: Slightly poor foam quality.
1: Poor foam quality.

### <Possibility of nozzle clogging of the foam discharge container>

5 g of the sample was dropped into a petri dish with a diameter of 10 cm, and left in an environment of 30°C and 20% without a lid for 24 hours. The state of the sample after 24 hours was evaluated according to the following criteria.

1: The whole sample is in a fluid state (no crystals have been precipitated), and the whole sample flows when the bottom of the petri dish is tilted vertically relative to the ground.

2: The part with fluidity is mixed with crystals, and the part with fluidity flows when the bottom of the petri dish is tilted vertically relative to the ground.

3: The part with fluidity is mixed with crystals, and the part with fluidity does not flow when the bottom of the petri dish is tilted vertically relative to the ground, but if the cigarette paper is pressed on the surface of the sample, the liquid with fluidity adheres to the cigarette paper.

4: There is no part with fluidity in the sample at all, or the part with fluidity does not flow when the bottom of the petri dish is tilted vertically relative to the ground and the liquid with fluidity does not adhere to the cigarette paper even if the cigarette paper is pressed on the surface of the sample.

**[Table 5]**

| | Example 33 | Example 34 | Example 35 | Example 36 | Example 37 | Example 38 | Example 39 | Example 40 |
|---|---|---|---|---|---|---|---|---|
| (A1-1) | 6 | 6 | 6 | 6 | 6 | 6 | 6 | 6 |
| (A1-3) | 6 | 6 | 6 | 6 | 6 | 6 | 6 | 6 |
| (B-1) | 6 | 6 | 6 | 6 | 6 | 6 | 6 | 6 |
| (C-1) | 5 | 10 | 15 | 20 | 25 | 30 | 40 | 45 |
| pH regulator*² | q.s. | q.s. | q.s. | q.s. | q.s. | q.s. | q.s. | q.s. |
| Water | Balance | Balance | Balance | Balance | Balance | Balance | Balance | Balance |
| Total | 100 | 100 | 100 | 100 | 100 | 100 | 100 | 100 |
| The content of component (A) | 3.3 | 3.3 | 3.3 | 3.3 | 3.3 | 3.3 | 3.3 | 3.3 |
| The content of component (A) in terms of acid | 2.91 | 2.91 | 2.91 | 2.91 | 2.91 | 2.91 | 2.91 | 2.91 |
| The content of component (B) | 6 | 6 | 6 | 6 | 6 | 6 | 6 | 6 |
| The content of component (C) | 5 | 10 | 15 | 20 | 25 | 30 | 40 | 45 |
| Component (B)/Component (A)(in terms of acid) | 2.1 | 2.1 | 2.1 | 2.1 | 2.1 | 2.1 | 2.1 | 2.1 |
| Component (C)/Component (B) | 0.83 | 1.67 | 2.50 | 3.33 | 4.17 | 5.00 | 6.67 | 7.50 |
| Detergency against pores | 5 | 5 | 5 | 5 | 5 | 5 | 3 | 3 |
| Foam quality | 4.0 | 4.0 | 4.0 | 4.0 | 4.0 | 3.0 | 3.0 | 3.0 |
| Possibility of clogging | 3 | 2 | 2 | 2 | 2 | 2 | 2 | 1 |

| | | | | | | | | |
|---|---|---|---|---|---|---|---|---|
| *2: 50% of liquid malic acid or 48% of potassium hydroxide was used as a pH regulator | | | | | | | | |

**[Table 6]**

| | Example 41 | Example 42 | Example 43 | Example 44 | Example 45 | Example 46 | Example 47 |
|---|---|---|---|---|---|---|---|
| (A1-1) | 6 | 6 | 6 | 6 | 6 | 6 | 6 |
| (A1-3) | 6 | 6 | 6 | 6 | 6 | 6 | 6 |
| (B-1) | 15 | 4.5 | 4 | 3 | 2 | 1 | 0.5 |
| (C-1) | 12.5 | 45 | 45 | 45 | 45 | 45 | 22.5 |
| pH regulator*² | q.s. | q.s. | q.s. | q.s. | q. s. | q.s. | q.s. |
| Water | Balance | Balance | Balance | Balance | Balance | Balance | Balance |
| Total | 100 | 100 | 100 | 100 | 100 | 100 | 100 |
| The content of component (A) | 3.3 | 3.3 | 3.3 | 3.3 | 3.3 | 3.3 | 3.3 |
| The content of component (A) in terms of acid | 2.91 | 2.91 | 2.91 | 2.91 | 2.91 | 2.91 | 2.91 |
| The content of component (B) | 15 | 4.5 | 4 | 3 | 2 | 1 | 0.5 |
| The content of component (C) | 12.5 | 45 | 45 | 45 | 45 | 45 | 22.5 |
| Component (B)/Component (A)(in terms of acid) | 5.2 | 1.5 | 1.4 | 1.0 | 0.7 | 0.3 | 0.2 |
| Component (C)/Component (B) | 0.83 | 10.00 | 11.25 | 15.00 | 22.50 | 45.00 | 45.00 |
| Detergency against pores | 3 | 3 | 3 | 3 | 3 | 3 | 3 |
| Foam quality | 4.3 | 3.0 | 3.0 | 3.0 | 3.0 | 3.0 | 3.5 |
| Possibility of clogging | 2 | 1 | 1 | 1 | 1 | 1 | 1 |

**[Table 7]**

| | Example 48 | Example 49 | Example 50 | Example 51 | Example 52 | Example 53 | Example 54 | Example 55 | Example 56 | Example 57 | Example 58 |
|---|---|---|---|---|---|---|---|---|---|---|---|
| (A1-1) | 6 | 6 | 6 | 6 | 6 | 6 | 6 | 6 | 6 | 6 | 6 |
| (A1-3) | 6 | 6 | 6 | 6 | 6 | 6 | 6 | 6 | 6 | 6 | 6 |
| (B-1) | 6 | 6 | 6 | 6 | 6 | 6 | 6 | 6 | 6 | 6 | 6 |
| (C-1) | 15.0 | | | | | | | | | | |
| (C-2) | | 15.0 | | | | | | | | | |
| (C-3) | | | 15.0 | | | | | | | | |
| (C-4) | | | | 15.0 | | | | | | | |
| (C-5) | | | | | 15.0 | | | | | | |
| (C-6) | | | | | | 15.0 | | | | | |
| (C-7) | | | | | | | 15.0 | | | | |
| (C-8) | | | | | | | | 15.0 | | | |
| (C-9) | | | | | | | | | 21.43 | | |
| (C-10) | | | | | | | | | | 15.0 | |
| (C-11) | | | | | | | | | | | 15.0 |
| pH regulator*² | q.s. | q.s. | q.s. | q.s. | q.s. | q.s. | q.s. | q.s. | q.s. | q.s. | q.s. |
| Water | Balance | Balance | Balance | Balance | Balance | Balance | Balance | Balance | Balance | Balance | Balance |
| Total | 100 | 100 | 100 | 100 | 100 | 100 | 100 | 100 | 100 | 100 | 100 |
| The content of component (A) | 3.3 | 3.3 | 3.3 | 3.3 | 3.3 | 3.3 | 3.3 | 3.3 | 3.3 | 3.3 | 3.3 |
| The content of component (A) in terms of acid | 2.91 | 2.91 | 2.91 | 2.91 | 2.91 | 2.91 | 2.91 | 2.91 | 2.91 | 2.91 | 2.91 |
| The content of component (B) | 6 | 6 | 6 | 6 | 6 | 6 | 6 | 6 | 6 | 6 | 6 |
| The content of component (C) | 15 | 15 | 15 | 15 | 15 | 15 | 15 | 15 | 15 | 15 | 15 |
| Component (B)/Component (A)(in terms of acid) | 2.1 | 2.1 | 2.1 | 2.1 | 2.1 | 2.1 | 2.1 | 2.1 | 2.1 | 2.1 | 2.1 |
| Component (C)/Component (B) | 2.5 | 2.5 | 2.5 | 2.5 | 2.5 | 2.5 | 2.5 | 2.5 | 2.5 | 2.5 | 2.5 |
| Detergency against pores | 5 | 4 | 4 | 5 | 4 | 5 | 5 | 5 | 5 | 4 | 3 |
| Foam quality | 4.0 | 2.8 | 3.0 | 4.0 | 4.3 | 3.8 | 4.0 | 4.3 | 4.0 | 2.5 | 2.3 |
| Possibility of clogging | 2 | 2 | 2 | 2 | 3 | 3 | 2 | 2 | 3 | 2 | 2 |

**[Table 8]**

| | Example 59 | Example 60 | Example 61 |
|---|---|---|---|
| (A1-1) | 6 | 6 | 6 |
| (B-1) | 6 | | |
| (B-2) | | 6 | |
| (B-3) | | | 6 |
| (C-1) | 15 | 15 | 15 |
| pH regulator*² | q.s. | q.s. | q.s. |
| Water | Balance | Balance | Balance |
| Total | 100 | 100 | 100 |
| The content of component (A) | 1.5 | 1.5 | 1.5 |
| The content of component (A) in terms of acid | 1.34 | 1.34 | 1.34 |
| The content of component (B) | 6 | 6 | 6 |
| The content of component (C) | 15 | 15 | 15 |
| Component (B)/Component (A)(in terms of acid) | 4.5 | 4.5 | 4.5 |
| Component (C)/Component (B) | 2.5 | 2.5 | 2.5 |
| Detergency against pores | 5 | 5 | 4 |
| Foam quality | 3.3 | 3.3 | 3.3 |
| Possibility of clogging | 2 | 1 | 1 |

**[Table 9]**

| | Example 62 | Example 63 | Example 64 | Example 65 | Example 66 | Example 67 | Example 68 | Example 69 |
|---|---|---|---|---|---|---|---|---|
| (A1-1) | | | | | | 6 | 6 | 6 |
| (A1-2) | 5 | | | | | | | |
| (A1-3) | | 5 | | | | | | |
| (A1-4) | | | 6 | | | | | |
| (A2-1) | | | | 1.5 | | | | |
| (A3-1) | | | | | 1.5 | | | |
| (A'-1) | | | | | | 1.11 | | |
| (A'-2) | | | | | | | 3.70 | |
| (A'-3) | | | | | | | | 3.36 |
| (B-1) | 6 | 6 | 6 | 6 | 6 | 6 | 6 | 6 |
| (C-1) | 15.0 | 15.0 | 15.0 | 15.0 | 15.0 | 15.0 | 15.0 | 15.0 |
| pH regulator*² | q.s. | q.s. | q.s. | q.s. | q.s. | q.s. | q.s. | q.s. |
| Water | Balance | Balance | Balance | Balance | Balance | Balance | Balance | Balance |
| Total | 100 | 100 | 100 | 100 | 100 | 100 | 100 | 100 |
| The content of component (A) | 1.5 | 1.5 | 1.5 | 1.5 | 1.5 | 1.5 | 1.5 | 1.5 |
| The content of component (A) in terms of acid | 1.39 | 1.31 | 1.40 | 1.40 | 1.50 | 1.34 | 1.34 | 1.34 |
| The content of ionic surfactant(s) other than component (A) in terms of acid | 0 | 0 | 0 | 0 | 0 | 1.00 | 0.92 | 0.94 |
| The content of ionic surfactant(s) other than component (A) in terms of acid/The content of component (A) in terms of acid | 0 | 0 | 0 | 0 | 0 | 0.75 | 0.69 | 0.70 |
| The content of component (B) | 6 | 6 | 6 | 6 | 6 | 6 | 6 | 6 |
| The content of component (C) | 15.0 | 15.0 | 15.0 | 15.0 | 15.0 | 15.0 | 15.0 | 15.0 |
| Component (B)/Component (A)(in terms of acid) | 4.3 | 4.6 | 4.3 | 4.3 | 4.0 | 4.5 | 4.5 | 4.5 |
| Component (C)/Component (B) | 2.5 | 2.5 | 2.5 | 2.5 | 2.5 | 2.5 | 2.5 | 2.5 |
| Detergency against pores | 5 | 5 | 5 | 5 | 5 | 4 | 5 | 5 |
| Foam quality | 3.0 | 4.0 | 3.3 | 3.0 | 3.0 | 4.0 | 4.3 | 4.3 |
| Possibility of clogging | 2 | 2 | 2 | 2 | 1 | 2 | 2 | 2 |

**[Table 10]**

| | Example 70 | Example 71 | Example 72 | Example 73 | Example 74 | Example 75 |
|---|---|---|---|---|---|---|
| (A1-1) | 4 | 6 | 8 | 12 | 16 | 32 |
| (B-1) | 6 | 6 | 6 | 6 | 6 | 6 |
| (C-1) | 15.0 | 15.0 | 15.0 | 15.0 | 15.0 | 15.0 |
| pH regulator*² | q.s. | q.s. | q.s. | q.s. | q.s. | q.s. |
| Water | Balance | Balance | Balance | Balance | Balance | Balance |
| Total | 100 | 100 | 100 | 100 | 100 | 100 |
| The content of component (A) | 1.0 | 1.5 | 2.0 | 3.0 | 4.0 | 8.0 |
| The content of component (A) in terms of acid | 0.89 | 1.34 | 1.79 | 2.68 | 3.57 | 7.14 |
| The content of component (B) | 6 | 6 | 6 | 6 | 6 | 6 |
| The content of component (C) | 15.0 | 15.0 | 15.0 | 15.0 | 15.0 | 15.0 |
| Component (B)/Component (A)(in terms of acid) | 6.7 | 4.5 | 3.4 | 2.2 | 1.7 | 0.8 |
| Component (C)/Component (B) | 2.5 | 2.5 | 2.5 | 2.5 | 2.5 | 2.5 |
| Detergency against pores | 5 | 5 | 5 | 5 | 5 | 3 |
| Foam quality | 3.3 | 3.3 | 3.3 | 3.5 | 3.5 | 3.5 |
| Possibility of clogging | 2 | 2 | 2 | 2 | 2 | 2 |

As can be seen from above Tables 5 to 10, the skin cleanser compositions obtained in the above examples can achieve both detergency against pores and foam quality as well as preventing the foam discharge container from clogging, and thus can be applied to the foam discharge container.

**In** summary, the skin cleanser compositions of the present invention can achieve excellent detergency against pores, particularly detergency against keratotic plugs, as well as good foaming property and foam quality, and further, can inhibit the precipitation of crystals to prevent the clogging of the foam discharge container.

## Claims

1. A skin cleanser composition comprising the following component (A) and component (B),
component (A) is one or more anionic surfactant(s) selected from the following components (A1), (A2), and (A3),
component (A1) is an N-acyl amino acid represented by the following general formula (1), or a salt thereof, 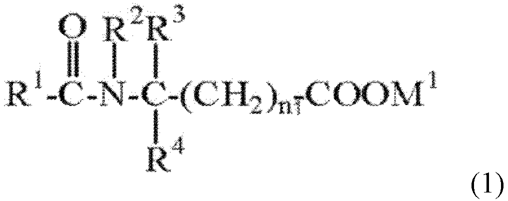
in formula (1), R¹ represents a linear or branched alkyl or alkenyl with 5 to 24 carbon atoms, R² represents hydrogen or an alkyl with 1 to 4 carbon atoms, R³ and R⁴ are the same or different and represent hydrogen, an alkyl with 1 to 4 carbon atoms, a hydroxyalkyl with 1 to 4 carbon atoms, or -(CH₂)ₘ₁-COOM², m1 and n1 are each independently a number from 0 to 20, M¹ and M² are the same or different and represent hydrogen, an alkali metal, an alkaline earth metal, a basic amino acid, ammonium or alkanolamine;
component (A2) is an N-acyl taurine represented by the following general formula (2), or a salt thereof,
R₁CO-NR₂-CH₂CH₂SO₃M (2)
in formula (2), R₁ represents a linear or branched alkyl or alkenyl with 8 to 20 carbon atoms, R₂ represents hydrogen or an alkyl with 1 to 3 carbon atoms, M represents hydrogen, an alkali metal, an alkaline earth metal, a basic amino acid, ammonium, or alkanolamine;
component (A3) is a fatty acid with 8 to 20 carbon atoms, or a salt thereof;
component (B) is one or more selected from 2-amino-2-hydroxymethyl-1,3-propanediol, 2-amino-2-methyl-1- propanol, 2-amino-2-methyl-1,3-propanediol, and monoethanolamine.

2. The skin cleanser composition of claim 1, wherein
when the component (A) comprises the component (A1), the content of the component (A1) in terms of acid is 0.5-15% by mass, and the mass ratio (B)/(A1) of the content of the component (B) to the content of the component (A1) in terms of acid is 0.5-10,
when the component (A) comprises the component (A2), the content of the component (A2) in terms of acid is 0.1-25% by mass, and the mass ratio (B)/(A2) of the content of the component (B) to the content of the component (A2) in terms of acid is 0.3-43,
when the component (A) comprises the component (A3), the content of the component (A3) in terms of acid is 1-8% by mass, and the mass ratio (B)/(A3) of the content of the component (B) to the content of the component (A3) in terms of acid is 1.5-7,
ionic surfactant(s) other than the component (A) may or may not be comprised, wherein the content of the anionic surfactant(s) other than the component (A), or the total content of the anionic surfactant(s) other than the component (A) and amphoteric surfactant(s) is less than 1.5% by mass in terms of acid.

3. The skin cleanser composition of claim 1 or 2, wherein
the content of the anionic surfactant(s) other than the component (A) or the total content of the anionic surfactant(s) other than the component (A) and amphoteric surfactant(s) is greater than 0% by mass in terms of acid,
the mass ratio of the content of the anionic surfactant(s) other than the component (A) in terms of acid or the total content of the anionic surfactant(s) other than the component (A) and the amphoteric surfactant(s) in terms of acid to the content of the component (A) in terms of acid, (the content of the anionic surfactant(s) other than the component (A) in terms of acid or the total content of the anionic surfactant(s) other than the component (A) and the amphoteric surfactant(s) in terms of acid)/(the content of the component (A) in terms of acid) is greater than 0 and less than 1.

4. The skin cleanser composition of any one of claims 1-3, wherein
the mass ratio (B)/(A1) of the content of the component (B) to the content of the component (A1) in terms of acid is 0.8-7.

5. The skin cleanser composition of any one of claims 1-4, wherein
the mass ratio (B)/(A1) of the content of the component (B) to the content of the component (A1) in terms of acid is 1.5-5.

6. The skin cleanser composition of any one of claims 1-5, wherein
the mass ratio (B)/(A2) of the content of the component (B) to the content of the component (A2) in terms of acid is 0.4-43.

7. The skin cleanser composition of any one of claims 1-6, wherein
the mass ratio (B)/(A2) of the content of the component (B) to the content of the component (A2) in terms of acid is 0.3-13.

8. The skin cleanser composition of any one of claims 1-7, wherein
the content of the component (B) is 0.08-35% by mass.

9. The skin cleanser composition of any one of claims 1-8, wherein
the skin cleanser composition is a foam discharge container-packed cleanser composition,
the skin cleanser composition further comprises (C) polyol(s),
component (B) is one or more selected from 2-amino-2-hydroxymethyl-1,3-propanediol, 2-amino-2-methyl-1- propanol, and 2-amino-2-methyl-1,3-propanediol, the mass ratio (C)/(B) of the content of the component (C) to the content of the component (B) is 0.7-60.

10. The skin cleanser composition of claim 9, wherein
the content of the component (C) is 3-60% by mass.

11. The skin cleanser composition of claim 9 or 10, wherein
the content of the component (B) is 0.3-20% by mass.

12. The skin cleanser composition of any one of claims 9-11, wherein
the total content of the component (B) and the component (C) is 10% by mass or more.

13. The skin cleanser composition of any one of claims 9-12, wherein
the component (C) is liquid at 25°C.

14. The skin cleanser composition of any one of claims 9-13, wherein
the component (C) is one or more selected from 1,2-propylene glycol, 1,3-propylene glycol, dipropylene glycol, polypropylene glycol, 1,3-butylene glycol, glycerol, diethylene glycol monoethyl ether, polyoxyalkylene glycerol ether, polyoxyalkylene diglycerol ether, polyoxyalkylene methyl glucoside, and sorbitol.

15. The skin cleanser composition of any one of claims 9-14, wherein
the gas-liquid mixing ratio of the foam discharge container is 10/1 to 38/1.

16. The skin cleanser composition of any one of claims 1-15, wherein
the pH value of the skin cleanser composition is 8.5-12.5 at 25°C.

17. The skin cleanser composition of any one of claims 1-16, wherein
the component (A1) is one or more selected from N-acyl glycine or a salt thereof, N-acyl alanine or a salt thereof, N-acyl threonine or a salt thereof, N-acyl serine or a salt thereof, N-acyl sarcosine or a salt thereof, N-acyl glutamic acid or a salt thereof, and N-acyl aspartic acid or a salt thereof.

18. The skin cleanser composition of any one of claims 1-17, wherein
the content of nonionic surfactant(s) is 10 % by mass or less.

19. A method for removing keratotic plugs, wherein
the skin cleanser composition of any one of claims 1-18 is applied to the skin, and after standing, the skin cleanser composition is rinsed off with water or wiped off with a wiping material.
